# EUROPÄISCHE PATENTSCHRIFT

(11) **EP 3 865 164 B1**
(45) Veröffentlichungstag und Bekanntmachung des Hinweises auf die Patenterteilung: **02.08.2023**
(21) Anmeldenummer: 21020044.0
(22) Anmeldetag: 02.02.2021
(51) Int. Cl.: A61M 16/00, A61M 16/10, A61B 5/00, A61M 16/06

(54) **ÜBERWACHUNGSSYSTEM ZUR ERKENNUNG VON LECKAGEN WÄHREND EINER BEATMUNG**
MONITORING SYSTEM FOR DETECTING LEAKAGE DURING VENTILATION
SYSTÈME DE SURVEILLANCE PERMETTANT DE DÉTECTER DES FUITES LORS D'UNE RESPIRATION

(30) Priorität: 14.02.2020 DE 102020104008
(43) Veröffentlichungstag der Anmeldung: 18.08.2021
(73) Patentinhaber: Löwenstein Medical Technology S.A., 2557 Luxembourg (LU)
(72) Erfinder: Schwaibold, Matthias, 76228 Karlsruhe (DE)
(74) Vertreter: Marx, Thomas

(56) Entgegenhaltungen:
- WO-A1-2012/126041
- WO-A2-2012/012835
- DE-A1-102007 039 004
- US-A1- 2010 186 741

## Beschreibung

Die vorliegende Erfindung betrifft ein Überwachungssystem zur Erkennung von Leckagen während einer Beatmung. Das Überwachungssystem umfasst wenigstens eine Überwachungseinrichtung zur Bestimmung einer Leckagerate aus wenigstens einem sensorisch erfassten Leckageparameter. Leckagen sind ein zentrales Problem in der nicht-invasiven Beatmung. Im Wesentlichen gibt es zwei Arten von Leckagen, die unterschiedliche Auswirkungen besitzen und unterschiedlich behoben werden können: Maskenleckagen und Mundleckagen. Die Maskenleckage kann meist dadurch behoben werden, dass die Maske bzw. der Sitz der Maske korrigiert werden. Die Mundleckage kann in der Regel durch das Schließen des Mundes behoben werden. Zudem gibt es auch noch sogenannte gewollte Leckagen, welche beispielsweise dem Ausspülen der ausgeatmeten Luft aus der Maske dienen.

Die Erkennung einer Leckage und ihrer Stärke bzw. Leckagerate ist sehr entscheidend für den Erfolg der Beatmung. Wichtig ist dabei auch eine Erkennung der Leckageart und besonders die Differenzierung der beiden oben genannten Leckagearten. Die Kenntnis über die Leckageart ist meist essentiell für eine optimale Korrektur der Leckage und die passende Versorgung des Patienten. In der Regel ist dies nur durch stationäre Aufenthalte und zusätzliche Sensoren oder visuelle Beobachtung des Patienten möglich.

Die Leckagen haben in der Regel auch einen entscheidenden Einfluss auf den Energieverbrauch und oft auch auf die Geräuschemissionen des Geräts. Zum Ausgleichen der Leckageverluste ist meist eine erhöhte Lüfterdrehzahl nötig. Das führt zu einem höheren Stromverbrauch. Besonders bei mobilen Geräten ist das von großem Nachteil, da sich die Akkulaufzeit erheblich verringern kann. Zudem ergeben sich meist erhöhte Lüftergeräusche. Das wird zum Beispiel bei der Schlaftherapie als unangenehm empfunden. WO 2012/012835 A2 offenbart ein automatisiertes Verfahren zur Erkennung von Mundleckagen, wobei zwischen einer kontinuierlichen Mundöffnung und einer periodisch wiederkehrenden Mundöffnung unterschieden werden kann. US 2010/186741 A1 beschreibt ein Verfahren und eine Vorrichtung zur Detektion von Mundleckagen während einer CPAP-Beatmung, woraufhin der Beatmungsdruck angepasst werden kann, um die Beatmung für den Patienten angenehmer zu machen. WO 2012/126041 A1 offenbart automatisierte Methoden für die Bewertung einer Beatmung, insbesondere für die Erkennung von Beatmungsinsuffizienzen, um eine Hypoventilation unter CPAP-Therapien zu vermeiden.

Demgegenüber ist es die Aufgabe der vorliegenden Erfindung, die Handhabung solcher Leckagen während der Beatmung zu verbessern. Insbesondere soll eine unaufwendige und zugleich besonders zuverlässige Erkennung der Leckageart möglich sein. Vorzugsweise soll auch unerfahrenen Benutzern bzw. Patienten eine zuverlässige Erkennung und Beseitigung von Leckagen ermöglicht werden. Vorzugsweise soll die Lösung auch im Bereich der Telemedizin bzw. in der Heimbeatmung vorteilhaft einsetzbar sein.

Diese Aufgabe wird gelöst mit einem Überwachungssystem des Anspruchs 1. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Das erfindungsgemäße Überwachungssystem dient zur Erkennung von Leckagen während einer Beatmung mit wenigstens einem Beatmungsgerät. Das Überwachungssystem umfasst wenigstens eine Überwachungseinrichtung zur insbesondere automatischen Bestimmung einer Leckagerate aus wenigstens einem sensorisch erfassten Leckageparameter. Dabei ist die Überwachungseinrichtung dazu geeignet und ausgebildet, wenigstens einen zeitlichen Verlauf des wenigstens einen Leckageparameters zu registrieren und wenigstens ein Maß für eine Änderungsrate des Leckageparameters im zeitlichen Verlauf zu bestimmen. Die Überwachungseinrichtung ist dazu geeignet und ausgebildet, in Abhängigkeit des Maßes wenigstens eine Mundleckage zu erkennen bzw. als eine solche zu identifizieren und vorzugsweise dadurch von wenigstens einer anderen Leckageart zu unterscheiden, wobei die Überwachungseinrichtung dazu geeignet und ausgebildet ist, als wenigstens eine andere Leckageart eine Maskenleckage zu erkennen und wenigstens eine Mundleckage und eine Maskenleckage voneinander zu unterscheiden.

Das erfindungsgemäße Überwachungssystem bietet viele Vorteile. Einen erheblichen Vorteil bietet die automatische Erkennung der Leckageart. Dadurch wird eine Mundleckage zuverlässig und einfach erkannt. So können auch unerfahrene Benutzer feststellen, dass eine Mundleckage vorliegt und diese durch entsprechende Maßnahmen beseitigen. Stationäre Aufenthalte und zeitaufwendige und kostenintensive Beobachtung des Patienten können so vermieden oder zumindest reduziert werden. Die Erfindung bietet auch in der Heimbeatmung bzw. Telemedizin ein zuverlässiges und komfortables Auffinden von Leckagen und deren Ursachen.

Durch das automatische Erkennen und die besonders einfache Beseitigung der Leckagen wird zudem auch der Energieverbrauch erheblich reduziert. Dadurch können die Akkulaufzeit verlängert oder durch den Einsatz kleinerer Akkus das Gewicht und die Größe der Geräte reduziert werden. Zudem werden unerwünschte Geräuschemissionen des Geräts vermeiden, die sonst bei der Kompensation der Leckageverluste auftreten. Es ist bevorzugt und vorteilhaft, dass die Überwachungseinrichtung dazu geeignet und ausgebildet ist, an dem Maß für die Änderungsrate ein Vorliegen eines abrupten Leckagerückgangs zu erkennen. Insbesondere ist die Überwachungseinrichtung dazu geeignet und ausgebildet, eine Mundleckage wenigstens in Abhängigkeit davon anzunehmen, dass ein abrupter Leckagerückgang vorliegt. Das Heranziehen solcher abrupter Leckagerückgänge bietet eine besonders zuverlässige Möglichkeit zur automatischen Erkennung von Mundleckagen. Die Überwachungseinrichtung nimmt insbesondere eine Mundleckage an, wenn ein abrupter Leckagerückgang vorliegt. Das Vorliegen eines abrupten Leckagerückgangs ist insbesondere an dem Maß für die Änderungsrate erkennbar. Insbesondere zeigt das Maß für die Änderungsrate dann einen besonders kräftigen und zugleich besonders zügigen Rückgang der Leckage. Insbesondere geht die Leckagerate abrupt und erheblich zurück.

Vorzugsweise liegt ein abrupter Leckagerückgang vor, wenn die Leckagerate innerhalb eines definierten Zeitraums um mindestens 10 % und insbesondere mindestens 20 % und vorzugsweise mindestens 30 % zurückgeht. Die Leckagerate kann innerhalb des definierten Zeitraums auch um mindestens 5 % oder 15 % oder 40 % oder auch 50 % oder 100 % oder 150 % oder 200 % oder 300 % zurückgehen, damit im Rahmen der vorliegenden Erfindung ein abrupter Leckagerückgang vorliegt. Solche Rückgänge sind besonders charakteristisch für das Beenden von Leckagen durch das Schließen des Mundes.

Vorzugsweise liegt ein abrupter Leckagerückgang vor, wenn die Leckagerate innerhalb von maximal acht Atemzügen und vorzugsweise maximal sechs Atemzügen und besonders bevorzugt maximal vier Atemzügen um das definierte Maß zurückgeht. Die Leckagerate kann auch innerhalb von maximal sieben Atemzügen oder maximal fünf Atemzügen oder auch maximal drei Atemzügen um das definierte Maß zurückgehen, damit ein abrupter Leckagerückgang vorliegt. Möglich ist auch, dass die Leckagerate innerhalb von maximal zwei Atemzügen oder während nur eines Atemzuges zurückgehen muss. Eine solche Bezugnahme zu den Atemzügen hat sich für die Bestimmung von abrupten Leckagerückgängen als besonders vorteilhaft erwiesen.

Es ist möglich, dass die Überwachungseinrichtung dazu geeignet und ausgebildet ist, eine Kennzahl für die Häufigkeit von abrupten Leckagerückgängen pro Zeiteinheit und insbesondere pro Stunde zu ermitteln und auszugeben. Die Kennzahl kann die Häufigkeit von abrupten Leckagerückgängen auch über mehrere Stunden oder den gesamten Therapiezeitraum ermitteln und angeben. Solche Kennzahlen ermöglichen eine sehr zügige und zugleich verlässliche Aussage über die Beatmungsqualität hinsichtlich der Leckage.

In allen Ausgestaltungen ist es besonders bevorzugt, dass die Überwachungseinrichtung dazu geeignet und ausgebildet ist, eine Mundleckage wenigstens in Abhängigkeit davon zu erkennen, ob sich der wenigstens eine Leckageparameter innerhalb wenigstens eines definierten Zeitraums um wenigstens ein definiertes Maß ändert und sich insbesondere verringert. Dann wird insbesondere ein abrupter Leckagerückgang angenommen. Eine solche Veränderung des Leckageparameters kann als eine notwendige Bedingung oder auch als eine hinreichende Bedingung für die Erkennung einer Mundleckage herangezogen werden. Ob sich der Leckageparameter innerhalb des definierten Zeitraums um das definierte Maß verändert hat, erkennt die Überwachungseinrichtung insbesondere aus dem Maß für die Änderungsrate des Leckageparameters. Beispielsweise ist das Maß für die Änderungsrate eine Steigung und/oder eine Ableitung und/oder eine andere Größe der Funktionsanalyse.

Der definierte Zeitraum beträgt insbesondere maximal 30 Sekunden und vorzugsweise maximal 15 Sekunden und besonders bevorzugt maximal 10 Sekunden. Möglich und vorteilhaft ist auch, dass der definierte Zeitraum maximal 8 Sekunden oder maximal 5 Sekunden oder maximal 3 Sekunden oder maximal 2 Sekunden oder auch maximal nur 1 Sekunde beträgt. Insbesondere beträgt der definierte Zeitraum zwischen drei und 30 Sekunden. Solche Zeiträume haben sich als besonders verlässlich für die Identifizierung von Mundleckagen erwiesen.

Der Leckageparameter betrifft insbesondere einen Fluss einer die Atmung betreffenden Gasströmung. Der Fluss kann auch als Volumenstrom oder Flow bezeichnet werden. Insbesondere ist der Leckageparameter ein Fluss. Insbesondere beschreibt der Leckageparameter, wie viel Volumen Atemgas pro Zeiteinheit durch die Leckage (also beispielsweise aus dem Mund oder zwischen Maske und Gesicht) entweicht. Der Leckageparameter kann auch den Druck einer die Atmung betreffenden Gasströmung betreffen. Dann sind insbesondere definierte Maße für die Änderung des Drucks hinterlegt, um eine Zuordnung zu bestimmten Leckagearten zu ermöglichen.

Ein definiertes Maß für die Änderung des Flusses beträgt insbesondere mindestens 10 l/min und vorzugsweise mindestens 15 l/min und besonders bevorzugt mindestens 25 l/min. Das definierte Maß kann auch mindestens 5 l/min oder mindestens 8 l/min oder mindestens 12 l/min oder mindestens 18 l/min oder mindestens 20 l/min betragen. Solche Änderungen des Flusses haben sich als besonders verlässlich zur Erkennung von Mundleckagen erwiesen.

Die Überwachungseinrichtung kann dazu geeignet und ausgebildet sein, die Leckageart dadurch zu erkennen, ob der Fluss oder ein statistischer Wert des Flusses, beispielsweise ein Mittelwert, oberhalb eines Grenzwerts liegt. Es konnte beobachtet werden, dass bei einer Mundleckage der Fluss unter bestimmten Bedingungen höher liegt als beispielsweise bei Maskenleckagen.

In einer vorteilhaften Ausgestaltung ist die Überwachungseinrichtung dazu geeignet und ausgebildet, einen Zeitraum im zeitlichen Verlauf des Leckageparameters für die Erkennung der Leckageart nicht zu berücksichtigen und/oder nur gewichtet zu berücksichtigen, wenn die Leckagerate nach dem abrupten Leckagerückgang (also nach der Änderung des Leckageparameters innerhalb des definierten Zeitraums um das definierte Maß) noch oberhalb eines Grenzwerts und insbesondere oberhalb von 25 l/min liegt. Als Grenzwert kann auch 30 l/min oder 35 l/min oder 40 l/min oder mehr vorgesehen sein. Der Grenzwert kann auch 20 l/min oder 15 l/min betragen. Das bietet eine besonders reproduzierbare Erkennung der Leckageart.

Die Überwachungseinrichtung ist vorzugsweise dazu geeignet und ausgebildet, die Erkennung der Leckageart nicht auszugeben und/oder mit wenigstens einem Warnhinweis auszugeben, wenn die Leckagerate (z. B. im Mittel und/oder über einen definierten Zeitraum) 50 l/min oder mehr beträgt. Es kann dazu auch ein Wert von wenigstens 30 l/min oder 40 l/min oder 60 l/min vorgesehen sein. Möglich ist, dass bei einer derart hohen Leckagerate wenigstens ein Alarm ausgegeben wird. Möglich ist auch, dass die Überwachungseinrichtung die während einer Zeit mit derart hohen Leckageraten erfassten Leckageparameter verwirft oder nur gewichtet berücksichtigt.

In einer besonders vorteilhaften Weiterbildung ist die Überwachungseinrichtung dazu geeignet und ausgebildet, für die Erkennung der Leckageart und insbesondere für die Erkennung der Mundleckage auch wenigstens zu berücksichtigen, wie häufig abrupte Leckagerückgänge auftreten und/oder wie regelmäßig abrupte Leckagerückgänge auftreten. Häufigkeit und Regelmäßigkeit sind besonders charakteristische Merkmale von Mundleckagen. Insbesondere berücksichtigt die Überwachungseinrichtung ein Erreichen einer Mindestgrenze für die Häufigkeit und/oder Regelmäßigkeit als wenigstens eine notwendige Bedingung für das Vorliegen einer Mundleckage. Ein Unterschreiten einer solchen Mindestgrenze wird insbesondere als eine notwendige Bedingung für ein Vorliegen einer Maskenleckage herangezogen.

In einer vorteilhaften Ausgestaltung ist die Überwachungseinrichtung dazu geeignet und ausgebildet, den zeitlichen Abstand eines abrupten Leckagerückgangs zu einem zeitlich zurückliegenden abrupten Leckagerückgang zu ermitteln. Vorzugsweise berücksichtigt die Überwachungseinrichtung für die Erkennung der Mundleckage auch wenigstens, wie groß der Abstand zwischen aufeinanderfolgenden abrupten Leckagerückgängen ist. Insbesondere wird ein Zeitabstand unterhalb eines Grenzwerts als eine notwendige Bedingung für die Erkennung einer Mundleckage herangezogen. Beispielsweise liegen abrupte Leckagerückgänge bei einer Mundleckage nur wenige Minuten oder wenige Sekunden und/oder wenige Atemzüge auseinander.

Vorzugsweise ist die Überwachungseinrichtung dazu geeignet und ausgebildet, für die Erkennung der Mundleckage auch Aufwachereignisse des Patienten zu berücksichtigen und insbesondere eine zeitliche Überlappung eines Aufwachereignisses mit dem Beginn eines abrupten Leckagerückgangs als wenigstens eine notwendige Bedingung für das Vorliegen einer Mundleckage zu berücksichtigen. Das Aufwachereignis kann sensorisch durch das Überwachungssystem und/oder über ein damit gekoppeltes Gerät erfasst werden. Es hat sich gezeigt, dass Aufwachereignisse besonders häufig mit einem Schließen des offenen Mundes einhergehen und somit auch häufig abrupte Leckagerückgänge mit sich bringen.

Es ist möglich und bevorzugt, dass die Überwachungseinrichtung dazu geeignet und ausgebildet ist, die nach dem Verschließen einer erkannten Mundleckage noch verbliebene Leckagerate zur Bestimmung einer gewollten Leckagerate und/oder einer Leckagerate einer anderen Art heranzuziehen. Insbesondere wird dazu die am Ende und/oder am Minimum des abrupten Leckagerückgangs noch verbliebene Leckagerate berücksichtigt. Zur Bestimmung der gewollten Leckagerate wird insbesondere auch ein Spülfluss bzw. Spülflow berücksichtigt.

Es ist auch möglich und bevorzugt, dass die Überwachungseinrichtung dazu geeignet und ausgebildet ist, eine Stärke der Mundleckage, insbesondere den Anteil der Mundleckage an der Leckagerate, über eine Höhe des Rückgangs der Leckagerate durch das Schließen des Mundes abzuleiten. Beispielsweise kann die Höhe des Rückgangs als Differenz der Leckagerate vor und nach Beendigung der Mundleckage ermittelt werden.

Die Überwachungseinrichtung kann dazu geeignet und ausgebildet sein, eine erkannte Mundleckage als wenigstens eine notwendige und/oder hinreichende Bedingung zur Erkennung eines Aufwachereignisses des Patienten heranzuziehen. Eine solche Ausgestaltung ist besonders vorteilhaft, wenn mit dem Überwachungssystem auch eine Überwachung des Schlafverhaltens des Patienten erfolgt. Es hat sich gezeigt, dass beim Aufwachen bzw. bei einem Arousal in der Regel ein Schließen des Mundes erfolgt.

In allen Ausgestaltungen ist die Überwachungseinrichtung dazu geeignet und ausgebildet, als wenigstens eine andere Leckageart eine Maskenleckage zu erkennen und eine Mundleckage und eine Maskenleckage voneinander zu unterscheiden.

Die Überwachungseinrichtung ist dazu geeignet und ausgebildet, die Maskenleckage wenigstens durch eine Dauer der Aufrechterhaltung einer definierten Leckagerate von der Mundleckage zu unterscheiden. Die Überwachungseinrichtung kann die Maskenleckage auch wenigstens durch eine Anzahl von definierten Rückgängen der Leckagerate und insbesondere durch eine Anzahl von abrupten Leckagerückgängen von der Mundleckage TCFunterscheiden. Das bietet eine besonders verlässliche Unterscheidung zwischen Mundleckagen und Maskenleckagen, da Maskenleckagen oft über einen langen Zeitraum und beispielsweise über mehrere Stunden oder während des gesamten Nachtschlafs nahezu unverändert aufrechterhalten werden.

Die Anmelderin behält sich vor, ein Überwachungssystem nach dem Oberbegriff von Anspruch 1 zu beanspruchen, bei dem die Überwachungseinrichtung dazu geeignet und ausgebildet ist, wenigstens einen zeitlichen Verlauf des Leckageparameters zu registrieren und wenigstens ein Maß für eine Änderungsrate des Leckageparameters im zeitlichen Verlauf zu bestimmen und in Abhängigkeit des Maßes wenigstens eine Maskenleckage zu erkennen.

Insbesondere ist die Überwachungseinrichtung dazu geeignet und ausgebildet, eine Erkennung der Leckageart während der Beatmung in bestimmten Zeitabständen und/oder fortlaufend durchzuführen und insbesondere erkannte Leckagearten zu protokollieren und vorzugsweise über wenigstens eine Anzeigeeinrichtung auszugeben. Insbesondere wird die erkannte Leckageart mit wenigstens einem weiteren Parameter ausgegeben. Beispielsweise wird zugleich die Anzahl der Mundleckagen und/oder die Leckagerate und/oder eine mittlere Dauer der Leckage oder dergleichen angezeigt.

In allen Ausgestaltungen ist es bevorzugt, dass die Überwachungseinrichtung dazu geeignet und ausgebildet ist, in Abhängigkeit einer erkannten Leckageart wenigstens eine Aktion auszuführen. Vorzugsweise umfasst die Aktion wenigstens eine Ausgabe wenigstens eins Warnhinweises und/oder wenigstens eines Alarms und/oder wenigstens eine Handlungsbeschreibung insbesondere zur Vermeidung von Leckagen.

In einer vorteilhaften Weiterbildung ist die Überwachungseinrichtung dazu geeignet und ausgebildet, das Beatmungsgerät in Abhängigkeit einer erkannten Leckageart anzusteuern und dabei wenigstens eine Steuergröße des Beatmungsgeräts anzupassen, insbesondere um eine Verringerung der Leckagerate und/oder der Leckagehäufigkeit zu erreichen. Es kann auch wenigstens ein Beatmungsparameter angepasst bzw. auf einen Sollwert geregelt werden.

Das Überwachungssystem kann wenigstens ein Beatmungsgerät umfassen. Das Beatmungsgerät ist insbesondere dazu geeignet und ausgebildet, von der Überwachungseinrichtung angesteuert zu werden. Insbesondere ist das Beatmungsgerät für eine Beatmung in der Art von CPAP und/oder APAP ausgebildet. Insbesondere ist das Beatmungsgerät dazu geeignet und ausgebildet, mittels wenigstens einer Beatmungseinrichtung wenigstens eine definierte Atemgasströmung zur Beatmung zu erzeugen.

Möglich ist, dass das Überwachungssystem separat zu dem Beatmungsgerät ausgebildet ist. Beispielsweise ist die Überwachungseinrichtung dazu in einem vom Beatmungsgerät separaten Gehäuse untergebracht und kann mit diesem über wenigstens eine Schnittstelle kabelgebunden und/oder drahtlos gekoppelt werden.

Offenbart wird auch ein Verfahren zum Betreiben eines Überwachungssystems, wie es zuvor beschrieben wurde. Ein derartiges Verfahren ist nicht Bestandteil der vorliegenden Erfindung.

Insbesondere ist das Verfahren so ausgebildet, dass die zuvor beschriebenen Aktionen der Überwachungseinrichtung ausgeführt werden. Insbesondere ist das zuvor beschriebene Überwachungssystem dazu geeignet und ausgebildet, nach dem erfindungsgemäßen Verfahren betrieben zu werden. Im Rahmen der vorliegenden Erfindung ist insbesondere vorgesehen, dass die Überwachungseinrichtung zur Ausführung der hier beschriebenen Verfahrensmerkmale bzw. Schritte geeignet und ausgebildet ist.

Die Mundleckage ist insbesondere dadurch bedingt, dass Atemgas durch einen während der Beatmung wenigstens teilweise geöffneten Mund entweicht. Die Mundleckage führt insbesondere zu einem Druckverlust und/oder Volumenverlust. Ein Schließen des Mundes führt in der Regel zu einem abrupten Leckagerückgang bezüglich der Mundleckage. Andere Leckagearten und beispielsweise eine Maskenleckage bleiben in der Regel nach dem Schließen des Mundes unverändert.

Eine Maskenleckage ist insbesondere durch eine ungewollte während der Beatmung aus einer Maske oder einer anderen Patientenschnittstelle austretende Atemgasströmung gekennzeichnet. In der Regel führt ein korrektes Anpassen der Maske zu einem messbaren Leckagerückgang bezüglich der Maskenleckage. Dabei erfolgt der Leckagerückgang jedoch signifikant weniger abrupt als durch das Verschließen des Mundes bei einer Mundleckage.

Der für eine Mundleckage charakteristische Leckagerückgang tritt insbesondere in einem Zeitraum auf, welcher für das Schließen des Mundes charakteristisch ist. Der für eine Maskenleckage charakteristische Leckagerückgang tritt insbesondere in einem solchen Zeitraum auf, wie er für das Anpassen der Maske durch den Patienten charakteristisch ist.

Es ist daher bevorzugt, dass der definierte Zeitraum für die Änderung des Leckageparameters bei einer Maskenleckage wenigstens um Faktor zwei oder wenigstens Faktor drei oder wenigstens Faktor vier oder wenigstens Faktor fünf oder auch wenigstens um Faktor zehn oder wenigstens Faktor 20 oder mehr größer ist als bei der Mundleckage.

Im Rahmen der vorliegenden Erfindung wird unter einer Leckage insbesondere stets eine ungewollte Leckage verstanden. Insbesondere ist im Rahmen der vorliegenden Erfindung bei der Leckagerate eine gewollte Leckage schon berücksichtigt. Beispielsweise wird ein Spülflow automatisch abgezogen.

Die Leckagerate gibt insbesondere eine Stärke der Leckage wieder. Die Leckagerate ist durch einen Volumenverlust und/oder Druckverlust definiert. Die Leckagerate wird aus dem wenigstens einen sensorisch erfassten Leckageparameter berechnet. Der Leckageparameter ist beispielsweise ein Fluss, auch als Flow oder Volumenstrom bezeichnet, ein Druck oder Druckverlauf und/oder eine Kombination solcher Größen.

Zur Erfassung des Leckageparameters ist insbesondere wenigstens ein Sensormittel vorgesehen. Das Sensormittel kann vom Überwachungssystem umfasst sein und/oder durch das Beatmungsgerät bereitgestellt werden. Beispielsweise weist das Beatmungsgerät wenigstens eine Sensoreinrichtung zur Steuerung bzw. Regelung der Beatmung auf, welche auch zur Erkennung der Leckageart eingesetzt werden kann. Beispielsweise ist ein Flowsensor und/oder Drucksensor oder dergleichen vorgesehen. Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
- Fig. 1: eine rein schematische Darstellung eines erfindungsgemäßen Überwachungssystems in einer perspektivischen Ansicht;
- Fig. 2: ein stark schematisiertes Schaubild mit Verläufen von Leckageparametern zur Erläuterung der Erkennung von Leckagen mittels der Erfindung; und
- Fig. 3-4: weitere stark schematisierte Schaubilder mit Verläufen von Leckageparametern zur Erläuterung der Erkennung von Leckagen mittels der Erfindung.

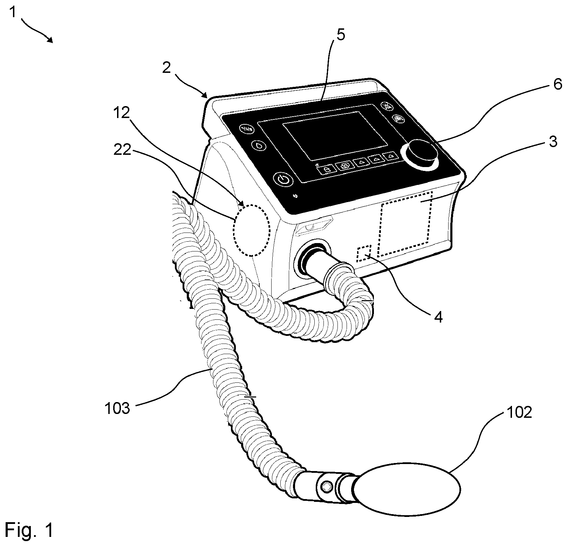

Die Figur 1 zeigt ein erfindungsgemäßes Überwachungssystem 1 mit einer Überwachungseinrichtung 3 zur Erkennung von Leckagen während einer nicht-invasiven Beatmung mit einem Beatmungsgerät 2. Das Beatmungsgerät 2 ist hier Teil des Überwachungssystems 1 und stellt die Einhausung der Überwachungseinrichtung 3 bereit. Die Überwachungseinrichtung 3 kann alternativ auch als separate Vorrichtung außerhalb des Beatmungsgeräts 2 angeordnet sein. Das hier gezeigte System 1 wird nach einem Verfahren betrieben. Das Überwachungssystem 1 ist hier als ein Heimbeatmungsgerät ausgebildet. Das Überwachungssystem 1 kann aber auch zur klinischen Beatmung eingesetzt werden.

Das Beatmungsgerät 2 weist im Inneren seines Gehäuses hier eine Beatmungseinrichtung 12 auf, welche mit einer Lüftereinrichtung 22 zur Erzeugung einer Atemgasströmung ausgestattet ist. Die Atemgasströmung wird über eine an die Beatmungseinrichtung 12 gekoppelte Schlaucheinrichtung 103 mit einer Atemmaske 102 dem Patienten zugeführt. Alternativ zur Atemmaske 102 können auch andere Patientenschnittstellen verwendet werden. Zusätzlich oder alternativ zur Lüftereinrichtung 22 kann auch eine Druckgasquelle vorgesehen sein.

Das Beatmungsgerät 2 umfasst hier eine Anzeigeeinrichtung 5 und eine Bedieneinrichtung 6. Dabei können auch Kombinationen von Bedieneinrichtung 6 und Anzeigeeinrichtung 5 vorgesehen sein, beispielsweise in der Art einer berührungsempfindlichen Anzeigefläche bzw. eines Touchscreens. Die Anzeigeeinrichtung 5 dient hier auch zur Darstellung von Informationen der Überwachungseinrichtung 3 und beispielsweise der Leckageart und der Leckagerate. Die Überwachungseinrichtung 3 kann ihre Informationen aber auch auf weiteren hier nicht gezeigten Anzeigeeinrichtungen ausgeben, z. B. auf einem Computerdisplay oder einem Tablet oder Smartphone oder dergleichen.

Die Beatmungseinrichtung 12 ist hier mit einem Sensormittel 4 wirkverbunden, welches ein oder mehrere Sensoren zur Erfassung von Beatmungsparametern und Leckageparametern und ggf. weiterer für die Beatmung charakteristischer Größen aufweist. Zum Beispiel umfasst das Sensormittel 4 einen hier nicht näher gezeigten Drucksensor, welcher die Druckverhältnisse der Atemgasströmung erfasst und/oder einen Flusssensor, welcher die Fluss bzw. den Flow erfasst.

Das Sensormittel 4 ist hier auch mit der Überwachungseinrichtung 3 wirkverbunden, sodass die erfassten Größen wenigstens teilweise auch von der Überwachungseinrichtung 3 verarbeitet werden können. Die Überwachungseinrichtung 3 kann auch eigene Sensormittel 4 umfassen. Zum Beispiel bestimmt die Überwachungseinrichtung 3 die Leckagerate aus wenigstens einem sensorisch erfassten Leckageparameter und z. B. einem Fluss.

Die Beatmungseinrichtung 12 umfasst hier eine nicht sichtbar innerhalb des Gehäuses angeordnete Steuereinrichtung zur Ansteuerung der Lüftereinrichtung 22. So können z. B. eine CPAP-Beatmung oder eine APAP-Beatmung ausgeführt werden. Für eine Beatmung wird die Beatmungseinrichtung 12 z. B. auf einen definierten Atemgasfluss und/oder ein Atemgasdruck eingestellt. Die Steuereinrichtung 12 kann einen notwendigen Minimaldruck bereitstellen und/oder Druckschwankungen kompensieren, die durch die Atemtätigkeit des Benutzers bedingt sind. Beispielsweise erfasst die Steuereinrichtung 12 mittels des Sensormittels 4 den gegenwärtigen Druck in der Atemmaske 102 und regelt die Leistung der Lüftereinrichtung 22 entsprechend nach, bis ein gewünschter Beatmungsdruck anliegt.

Zudem kann die Beatmungseinrichtung 12 hier auch von der Überwachungseinrichtung 3 angesteuert werden. Dazu ist die Überwachungseinrichtung 3 mit der Steuereinrichtung wirkverbunden.

Als Geräteparameter, die über die Erkennung der Leckagearten gesteuert werden können, gehören beispielsweise die nachfolgend beschriebenen.

Bei einer erkannten sehr großen Maskenleckage kann mindestens ein Therapiedruck-Niveau abgesenkt werden, um die Maskenleckage zu reduzieren.

Bei einer erkannten Mundleckage kann - bevorzugt in Kombination mit einer Auswertung des inspiratorischen Atemflussverlaufes - mindestens ein Therapiedruck-Niveau angehoben werden, um partielle Atemwegsobstruktionen zu therapieren, die zu einer erhöhten Atemanstrengung mit Mundöffnung geführt haben.

Bei einer erkannten Mundleckage kann die Leistung für die Befeuchtung und Erwärmung der Atemluft angehoben werden, um ein Austrocknen der Schleimhäute zu verhindern.

Über zusätzliche mit dem Gerät verbundene Sensoren kann die Genauigkeit der Differenzierung zwischen Maskenleckage und Mundleckage verbessert werden. Insbesondere durch automatische Auswertung von: Messung der Mundöffnung, z. B. über Dehnungsmess-Streifen in einem Kinnband oder Abstandssensoren, die oberhalb und unterhalb des Mundes angebracht werden; eine Bilderkennung aus einer Foto-/Videoaufzeichnung/IR-Aufnahmen des Patienten; Fluss-Sensor, z. B. Thermistor, der im Bereich der eventuellen Atemströmung vor dem Mund angebracht wird.

Über zusätzliche mit dem Gerät verbundene Sensoren kann die Relevanz einer Mund- oder Maskenleckage ermittelt werden. Z. B. mit einem SpO2-Sensor (Pulsoximeter) oder CO2-Sensor (z. B. ptCO2) und Auswertung der Blutgase in Phasen mit und ohne Leckagen.

Um Leckagen während der Beatmung zu erkennen und um verschiedene Arten von Leckagen unterscheiden zu können, wird hier wenigstens ein zeitlicher Verlauf eines oder mehrerer Leckageparameter registriert. Dann wird aus dem zeitlichen Verlauf wenigstens ein Maß für eine zeitliche Änderungsrate des Leckageparameters bestimmt. In Abhängigkeit des Maßes kann dann die Mundleckage von einer anderen Leckageart und z. B. von einer Maskenleckage unterschieden werden.

Mundleckagen sind dadurch gekennzeichnet, dass sie wiederholt unterbrochen werden durch einen plötzlichen Mundschluss, z. B. beim Schlucken, bei Arousals oder zyklisch bei schlafbezogenen Atemstörungen. Maskenleckagen enden dagegen weniger abrupt, typischerweise durch eine manuelle bessere Fixierung der Maske oder der Bänderung.

Dabei ist es ein Vorteil der Erfindung, dass abrupte Leckagerückgänge bzw. Leckageende-Vorgänge erkannt und deren Häufigkeit gezählt und als Indikator für das Vorliegen von Mundleckagen herangezogen werden können. In einer beispielhaften Ausführung wird das Leckageniveau nach einem abrupten Abfall der Leckage (Zustand bei gerade geschlossenem Mund) als Stärke der Maskenleckage geschätzt und ausgewiesen.

Zusätzlich kann die Stärke der Mundleckage über die Höhe des Rückgangs geschätzt werden. Wenn die Stärken der Mundleckage und Maskenleckage somit an mehreren Stützstellen im Laufe der Therapie geschätzt werden können, kann auch die gesamte Leckage der Therapie (zum Beispiel entweder Mittelwert oder Median oder gewichteter Mittelwert oder Perzentil) anteilig als Schätzung aufgeteilt werden in Gesamt-Stärke der Mundleckage und Maskenleckage.

Eine beispielhafte Ausführung sieht vor: Auswertung des Leckagesignals und Suche nach einem Einbruch des Signals um mindestens x l/min in höchstens y Sekunden, mit x im bevorzugten Bereich von 10 bis 40 und y im Bereich 3 bis 30.

Beispielsweise kann dann über eine Ausgabe am Gerät oder in einer begleitenden Software oder Tele-Überwachung nicht nur wie bisher die Stärke der Leckage ausgegeben werden, sondern auch ein Indikator für die Art der Leckage, also z. B. Maskenleckage oder Mundleckage oder beides parallel.

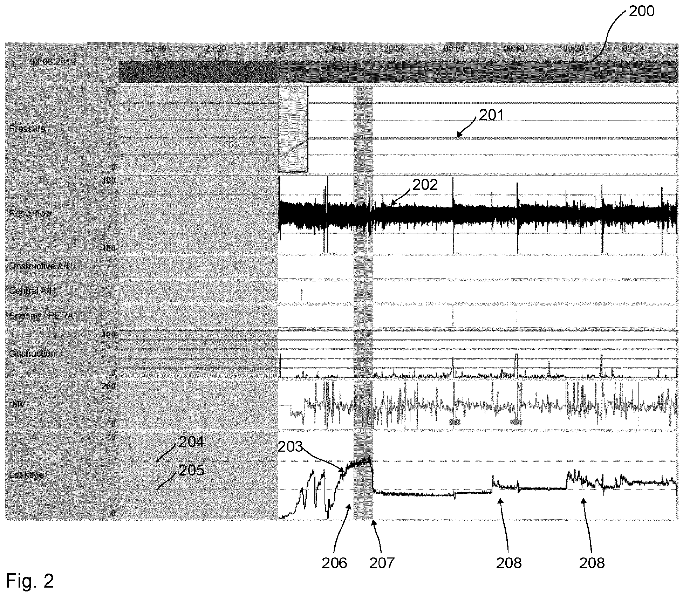

Die Figur 2 zeigt einen beispielhaften Beatmungsverlauf mit erfindungsgemäß erkannten Leckagearten. Dazu wurde hier eine Leckagerate 203 aufgezeichnet und deren zeitliche Änderungsrate ausgewertet. Die Leckagerate 203 entspricht hier einem Fluss in l/min. Die Leckagerate 203 und weitere Beatmungsparameter werden durch entsprechende Sensormittel 4 während der Beatmung erfasst und als Verläufe über die Zeit 200 registriert. Die Beatmungsparameter sind hier z. B. der Atemgasdruck 201 und der Atemgasfluss bzw. Flow 202 sowie weitere für die Beatmung hilfreiche Parameter.

Die Überwachungseinrichtung 3 ist so ausgebildet, dass sie aus der hier gezeigten Leckagerate 203 die Mundleckage 206 und die Maskenleckagen 208 erkennen kann.

Die Änderungsrate der Leckagerate 203 ändert sich hier über die Zeit 200. Ein besonders abrupter Leckagerückgang 207 ist hier mit dem Bezugszeichen 207 markiert. Zu dieser Zeit fällt die Leckagerate 203 innerhalb nur weniger Sekunden um mehr als 25 l/min ab. Es kann daher mit hoher Wahrscheinlichkeit angenommen werden, dass der Leckagerückgang 207 durch das Schließen des Mundes verursacht wurde. Die zuvor beobachtete Leckagerate 203 kann somit ebenfalls mit hoher Wahrscheinlichkeit auf eine Mundleckage 206 als Ursache zurückgeführt werden.

Die Leckagerate 203 sinkt hier durch das Schließen des Mundes hier um mehr als 15 l/min innerhalb von maximal 10 Sekunden ab, was zum Beispiel als Bedingung zur Erkennung Mundleckage 206 in der Überwachungseinrichtung 3 hinterlegt ist. Zudem ist für eine Erkennung einer Mundleckage vorgesehen, dass die Leckagerate 203 nach dem Schließen des Mundes auf maximal 25 l/min absinken muss, damit eine verlässliche Auswertung gewährleistet werden kann. Liegt die Leckagerate nach dem Leckagerückgang 207 noch oberhalb von 25 l/min, verwirft die Überwachungseinrichtung 3 dieses Ereignis.

Für die Erkennung der Mundleckage werden hier Grenzwerte 204, 205 berücksichtigt. Ein Überschreiten des Grenzwertes 204 kennzeichnet hier einen geöffneten Mund. Ein Unterschreiten des Grenzwertes 205 kennzeichnet hier einen geschlossenen Mund.

Die nach dem Leckagerückgang 207 erfasste Leckagerate 203 kann zudem als eine gewollte Leckagerate 203 bzw. eine übliche Hintergrundrate angenommen werden.

Anschließend nimmt die Leckagerate 203 hier wieder zu und wird unregelmäßiger. Dabei sind die Rückgänge der Leckagerate 203 jedoch nicht abrupt wie beim Schließen des Mundes, sondern verlaufen sehr langsam und steigen immer wieder an. Es kann hier von Maskenleckagen 208 ausgegangen werden. Hier ist gut zu erkennen, dass die Maskenleckage 208 nicht abrupt, sondern im Wesentlichen gar nicht endet. In der Regel endet die Maskenleckage 208 erst dann, wenn der Patient wach wird und die Maske 102 neu ausrichtet und fixiert.

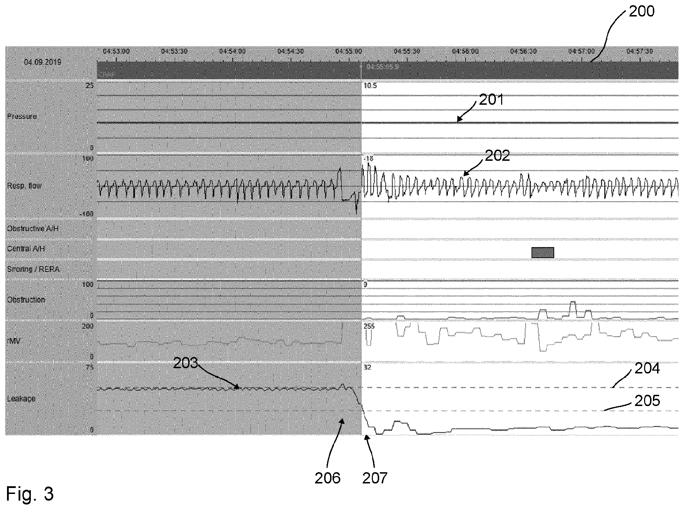

In der Figur 3 ist ein weiterer beispielhafter Beatmungsverlauf gezeigt. Auch hier zeigt die Leckagerate 203 einen sehr abrupten Leckagerückgang 207, welcher zur Identifizierung einer Mundleckage 206 führt. Der Mund stand während der Beatmung über mehrere Minuten offen und wurde dann geschlossen, sodass innerhalb weniger Sekunden die Leckagerate 203 signifikant abgesunken ist. Hier ist auch gut zu erkennen, dass die Mundleckage, im Gegensatz zu ihrem Ende, nicht abrupt, sondern langsam über einen längeren Zeitraum ansteigt. Nach dem Schließen des Mundes bleibt die Leckagerate 203 hier im Wesentlichen stabil, sodass hier keine Maskenleckage vorliegt.

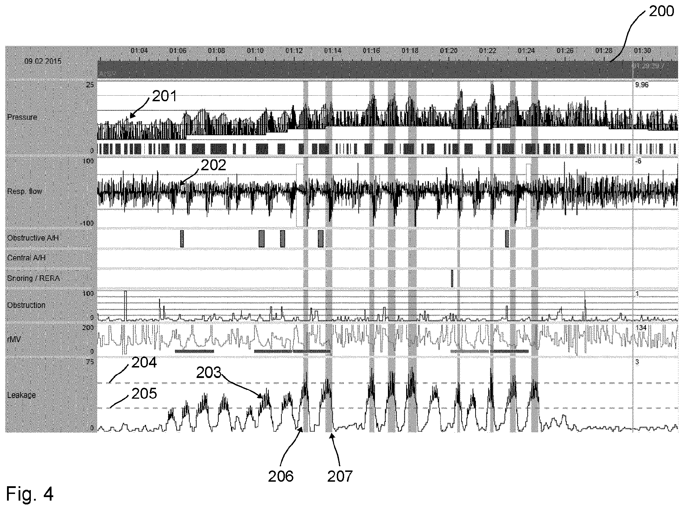

Die Figur 4 zeigt einen weiteren beispielhaften Verlauf einer Beatmung eines Patienten mit einer Atemstörung. Der Patient öffnet hier seinen Mund periodisch und besonders häufig, sodass das Öffnen des Mundes mit dem Atemmuster korrespondiert. Ein Schließen des Mundes erfolgt in Kombination mit Aufwachereignissen, welche sich beispielsweise aus den charakteristischen Spitzen im Verlauf Flusses 202 erkennen lassen. Hier ist besonders gut zu erkennen, dass beim Verschließen des Mundes abrupte Leckagerückgänge 207 auftreten. Zu einem Anstieg der Leckagerate 203 kommt es hier im Wesentlichen erst dann, wenn der Mund langsam wieder geöffnet wird. Besonders gut ist hier auch die Regelmäßigkeit des Auftretens von Mundleckagen 206 zu erkennen.

### Bezugszeichenliste:

- 1: Überwachungssystem
- 2: Beatmungsgerät
- 3: Überwachungseinrichtung
- 4: Sensormittel
- 5: Anzeigeeinrichtung
- 6: Bedieneinrichtung
- 12: Beatmungseinrichtung
- 22: Lüftereinrichtung
- 102: Atemmaske
- 103: Schlaucheinrichtung
- 200: Zeit
- 201: Druck
- 202: Fluss
- 203: Leckagerate
- 204: Grenzwert
- 205: Grenzwert
- 206: Mundleckage
- 207: abrupter Leckagerückgang
- 208: Maskenleckage

## Patentansprüche

1. Überwachungssystem (1) zur Erkennung von Leckagen während einer Beatmung mit wenigstens einem Beatmungsgerät (2), umfassend wenigstens eine Überwachungseinrichtung (3) zur Bestimmung einer Leckagerate aus wenigstens einem sensorisch erfassten Leckageparameter,
wobei die Überwachungseinrichtung (3) dazu geeignet und ausgebildet ist, wenigstens einen zeitlichen Verlauf des Leckageparameters zu registrieren und wenigstens ein Maß für eine Änderungsrate des Leckageparameters im zeitlichen Verlauf zu bestimmen und in Abhängigkeit des Maßes wenigstens eine Mundleckage zu erkennen, sodass eine Mundleckage von wenigstens einer anderen Leckageart unterschieden werden kann,
wobei die Überwachungseinrichtung (3) dazu geeignet und ausgebildet ist, als wenigstens eine andere Leckageart eine Maskenleckage zu erkennen und wenigstens eine Mundleckage und eine Maskenleckage voneinander zu unterscheiden, **dadurch gekennzeichnet,**
**dass** die Überwachungseinrichtung (3) dazu geeignet und ausgebildet ist, die Maskenleckage wenigstens durch eine Dauer der Aufrechterhaltung einer definierten Leckagerate von der Mundleckage zu unterscheiden und/oder durch eine Anzahl von definierten Rückgängen der Leckagerate zu unterscheiden
und **dass** die Leckagerate durch einen Volumenverlust und/oder Druckverlust definiert ist.

2. Überwachungssystem (1) nach dem vorhergehenden Anspruch, wobei die Überwachungseinrichtung (3) dazu geeignet und ausgebildet ist, an dem Maß für die Änderungsrate ein Vorliegen eines abrupten Leckagerückgangs zu erkennen und eine Mundleckage wenigstens in Abhängigkeit davon anzunehmen, dass ein abrupter Leckagerückgang vorliegt.

3. Überwachungssystem (1) nach dem vorhergehenden Anspruch, wobei ein abrupter Leckagerückgang vorliegt, wenn die Leckagerate innerhalb eines definierten Zeitraums um mindestens 10 % und insbesondere mindestens 20 % zurückgeht.

4. Überwachungssystem (1) nach einem der beiden vorhergehenden Ansprüche, wobei ein abrupter Leckagerückgang vorliegt, wenn die Leckagerate innerhalb von maximal 8 Atemzügen und vorzugsweise maximal 6 Atemzügen und besonders bevorzugt maximal 4 Atemzügen um wenigstens ein definiertes Maß zurückgeht.

5. Überwachungssystem (1) nach einem der drei vorhergehenden Ansprüche, wobei die Überwachungseinrichtung (3) dazu geeignet und ausgebildet ist, eine Kennzahl für die Häufigkeit von abrupten Leckagerückgängen pro Zeiteinheit und insbesondere pro Stunde zu ermitteln und auszugeben.

6. Überwachungssystem (1) nach einem der vorhergehenden Ansprüche, wobei die Überwachungseinrichtung (3) dazu geeignet und ausgebildet ist, eine Mundleckage wenigstens in Abhängigkeit davon zu erkennen, ob sich der wenigstens eine Leckageparameter innerhalb wenigstens eines definierten Zeitraums um wenigstens ein definiertes Maß ändert und insbesondere verringert, sodass ein abrupter Leckagerückgang angenommen werden kann.

7. Überwachungssystem (1) nach dem vorhergehenden Anspruch, wobei der definierte Zeitraum maximal 30 Sekunden und vorzugsweise maximal 15 Sekunden und besonders bevorzugt maximal 10 Sekunden beträgt.

8. Überwachungssystem (1) nach einem der beiden vorhergehenden Ansprüche, wobei der Leckageparameter einen Fluss einer die Atmung betreffenden Gasströmung betrifft und wobei ein definiertes Maß für die Änderung des Flusses mindestens 10 l/min und vorzugsweise mindestens 15 l/min und besonders bevorzugt mindestens 25 l/min beträgt.

9. Überwachungssystem (1) nach einem der vorhergehenden Ansprüche, wobei die Überwachungseinrichtung (3) dazu geeignet und ausgebildet ist, einen Zeitraum im zeitlichen Verlauf des Leckageparameters für die Erkennung der Leckageart nicht zu berücksichtigen und/oder nur gewichtet zu berücksichtigen, wenn die Leckagerate nach dem abrupten Leckagerückgang noch oberhalb eines Grenzwerts und insbesondere oberhalb von 25 l/min liegt.

10. Überwachungssystem (1) nach einem der vorhergehenden Ansprüche, wobei die Überwachungseinrichtung (3) dazu geeignet und ausgebildet ist, die Erkennung der Leckageart nicht auszugeben und/oder mit wenigstens einem Warnhinweis auszugeben, wenn die Leckagerate im Mittel und/oder über einen definierten Zeitraum 50 l/min oder mehr beträgt.

11. Überwachungssystem (1) nach einem der vorhergehenden Ansprüche, wobei die Überwachungseinrichtung (3) dazu geeignet und ausgebildet ist, für die Erkennung der Mundleckage auch wenigstens zu berücksichtigen, wie häufig abrupte Leckagerückgänge auftreten und/oder wie regelmäßig abrupte Leckagerückgänge auftreten.

12. Überwachungssystem (1) nach einem der vorhergehenden Ansprüche, wobei die Überwachungseinrichtung (3) dazu geeignet und ausgebildet ist, den zeitlichen Abstand eines abrupten Leckagerückgangs zu einem zeitlich zurückliegenden abrupten Leckagerückgang zu ermitteln und für die Erkennung der Mundleckage auch wenigstens zu berücksichtigen, wie groß der Abstand zwischen aufeinanderfolgenden abrupten Leckagerückgängen ist.

13. Überwachungssystem (1) nach einem der vorhergehenden Ansprüche, wobei die Überwachungseinrichtung (3) dazu geeignet und ausgebildet ist, für die Erkennung der Mundleckage auch wenigstens Aufwachereignisse des Patienten zu berücksichtigen und eine zeitliche Überlappung eines Aufwachereignisses mit dem Beginn eines abrupten Leckagerückgangs als wenigstens eine notwendige Bedingung für das Vorliegen einer Mundleckage zu berücksichtigen.

14. Überwachungssystem (1) nach einem der vorhergehenden Ansprüche, wobei die Überwachungseinrichtung (3) dazu geeignet und ausgebildet ist, die nach Verschließen einer erkannten Mundleckage noch verbliebene Leckagerate zur Bestimmung einer gewollten Leckagerate heranzuziehen.

15. Überwachungssystem (1) nach einem der vorhergehenden Ansprüche, wobei die Überwachungseinrichtung (3) dazu geeignet und ausgebildet ist, eine erkannte Mundleckage als wenigstens eine notwendige und/oder hinreichende Bedingung zur Erkennung eines Aufwachereignisses des Patienten heranzuziehen.

16. Überwachungssystem (1) nach einem der vorhergehenden Ansprüche, wobei die Überwachungseinrichtung (3) dazu geeignet und ausgebildet ist, die Maskenleckage wenigstens durch eine Anzahl von abrupten Leckagerückgängen zu unterscheiden.

17. Überwachungssystem (1) nach einem der vorhergehenden Ansprüche, wobei die Überwachungseinrichtung (3) dazu geeignet und ausgebildet ist, eine Erkennung der Leckageart während der Beatmung in bestimmten Zeitabständen und/oder fortlaufend durchzuführen und erkannte Leckagearten zu protokollieren und vorzugsweise über wenigstens eine Anzeigeeinrichtung (5) auszugeben.

18. Überwachungssystem (1) nach einem der vorhergehenden Ansprüche, wobei die Überwachungseinrichtung (3) dazu geeignet und ausgebildet ist, in Abhängigkeit einer erkannten Leckageart wenigstens eine Aktion auszuführen und wobei die Aktion eine Ausgabe wenigstens eines Warnhinweises und/oder Alarms und/oder einer Handlungsbeschreibung zur Vermeidung von Leckagen umfasst und wobei die Überwachungseinrichtung (3) dazu geeignet und ausgebildet ist, das Beatmungsgerät (2) in Abhängigkeit einer erkannten Leckageart anzusteuern und dabei wenigstens eine Steuergröße des Beatmungsgeräts (2) anzupassen, um eine Verringerung der Leckagerate und/oder der Leckagehäufigkeit zu erreichen.

19. Überwachungssystem (1) nach einem der vorhergehenden Ansprüche, umfassend wenigstens ein Beatmungsgerät (2).

## Claims

1. A monitoring system (1) for detecting leakages during ventilation with at least one ventilator (2), wherein the system comprises at least one monitoring device (3) for determining a leakage rate from at least one leakage parameter detected by one or more sensors,
the monitoring device (3) being suitable and configured for recording at least one time profile of the leakage parameter, for determining at least one measure for a rate of change of the leakage parameter in the time profile, and for detecting at least one mouth leakage in accordance with the at least one measure, such that a mouth leakage can be differentiated from at least one other leakage type, wherein the monitoring device (3) is suitable and configured for detecting a mask leakage as at least one other leakage type, and for differentiating at least a mouth leakage and a mask leakage from each other,
**characterized in**
**that** the monitoring device (3) is suitable and configured for differentiating the mask leakage from the mouth leakage at least by a duration for which a defined leakage rate is maintained, and/or for differentiating them by a number of defined reversals of the leakage rate,
and **that** the leakage rate is defined by a volume loss and/or pressure loss.

2. The monitoring system (1) of the preceding claim, wherein the monitoring device (3) is suitable and configured for detecting a presence of an abrupt leakage reversal using the measure for the rate of change, and for assuming a mouth leakage at least in accordance with an abrupt leakage reversal being present.

3. The monitoring system (1) of the preceding claim, wherein an abrupt leakage reversal is present when the leakage rate falls by at least 10% and in particular by at least 20% within a defined time period.

4. The monitoring system (1) of one of the two preceding claims, wherein an abrupt leakage reversal is present when the leakage rate falls by at least a defined measure within a maximum of eight breaths and preferably a maximum of six breaths and particularly preferably a maximum of four breaths.

5. The monitoring system (1) of one of the three preceding claims, wherein the monitoring device (3) is suitable and configured for determining and outputting a characteristic number for an incidence of abrupt leakage reversals per unit of time and in particular per hour.

6. The monitoring system (1) of one of the preceding claims, wherein the monitoring device (3) is suitable and configured for detecting a mouth leakage at least in accordance with whether the at least one leakage parameter changes by at least a defined measure within at least one defined time period, such that an abrupt leakage reversal can be assumed.

7. The monitoring system (1) of the preceding claim, wherein the defined time period is a maximum of 30 seconds and preferably a maximum of 15 seconds and particularly preferably a maximum of 10 seconds.

8. The monitoring system (1) of one of the two preceding claims, wherein the leakage parameter is a flow of a gas stream relating to the respiration, and wherein a defined measure for the change of the flow is at least 10 l/min and preferably at least 15 l/min and particularly preferably at least 25 l/min.

9. The monitoring system (1) of one of the preceding claims, wherein the monitoring device (3) is suitable and configured for not taking account and/or for taking only weighted account of a time period in the time profile of the leakage parameter for the detection of the leakage type when the leakage rate, after the abrupt leakage reversal, still lies above a limit value and in particular above 25 l/min.

10. The monitoring system (1) of one of the preceding claims, wherein the monitoring device (3) is suitable and configured for not outputting the detection of the leakage type and/or for outputting it with at least one warning when the leakage rate, on average and/or over a defined time period, is 50 l/min or more.

11. The monitoring system (1) of one of the preceding claims, wherein the monitoring device (3) is suitable and configured for detecting the mouth leakage by also at least taking account of how often abrupt leakage reversals occur and/or how regularly abrupt leakage reversals occur.

12. The monitoring system (1) of one of the preceding claims, wherein the monitoring device (3) is suitable and configured for determining a time difference between one abrupt leakage reversal and a past abrupt leakage reversal, and for detecting the mouth leakage by at least also taking account of how great a difference is between successive abrupt leakage reversals.

13. The monitoring system (1) of one of the preceding claims, wherein the monitoring device (3) is suitable and configured for detecting the mouth leakage by also at least taking account of arousal events on the part of a patient, and by considering a time overlap of an arousal event with a start of an abrupt leakage reversal as at least one necessary condition for the presence of a mouth leakage.

14. The monitoring system (1) of one of the preceding claims, wherein the monitoring device (3) is suitable and configured for determining a desired leakage rate by using a leakage rate that still remains after closure of a detected mouth leakage.

15. The monitoring system (1) of one of the preceding claims, wherein the monitoring device (3) is suitable and configured for using a detected mouth leakage as at least one necessary and/or sufficient condition for a detection of an arousal event on the part of a patient.

16. The monitoring system (1) of one of the preceding claims, wherein the monitoring device (3) is suitable and configured for differentiating the mask leakage at least by a number of abrupt leakage reversals.

17. The monitoring system (1) of one of the preceding claims, wherein the monitoring device (3) is suitable and configured for carrying out a detection of the leakage type during ventilation at defined time intervals and/or continuously, and for recording detected leakage types and preferably outputting them via at least one display device (5).

18. The monitoring system (1) of one of the preceding claims, wherein the monitoring device (3) is suitable and configured for executing at least one action in accordance with a detected leakage type, and wherein the action comprises an output of at least one warning and/or alarm and/or an action description for avoiding leakages, and wherein the monitoring device (3) is suitable and configured for actuating the ventilator (2) in accordance with a detected leakage type, and for adapting at least one control variable of the ventilator (2) in order to obtain a decrease in the leakage rate and/or the leakage incidence.

19. The monitoring system (1) of one of the preceding claims, comprising at least one ventilator (2).

## Revendications

1. Système de surveillance (1) permettant de détecter des fuites lors d'une respiration avec au moins un appareil respiratoire (2), comprenant au moins un dispositif de surveillance (3) pour la détermination d'un taux de fuite à partir d'au moins un paramètre de fuite détecté par capteur,
le dispositif de surveillance (3) étant adapté et conçu pour enregistrer au moins un déroulement temporel du paramètre de fuite et pour déterminer au moins un niveau pour un taux de modification du paramètre de fuite dans le déroulement temporel et, en fonction du niveau, pour détecter au moins une fuite buccale, de telle sorte qu'il est possible de distinguer entre une fuite buccale et au moins un autre type de fuite,
le dispositif de surveillance (3) étant adapté et conçu pour détecter une fuite de masque comme au moins un autre type de fuite et pour au moins faire la distinction entre une fuite buccale et une fuite de masque, **caractérisé en ce que**
le dispositif de surveillance (3) est adapté et conçu pour faire la distinction entre la fuite de masque et la fuite buccale au moins par une durée de maintien d'un taux de fuite défini et/ou par un nombre de baisses du taux de fuite définies,
et **en ce que** le taux de fuite est défini par une perte de volume et/ou une perte de pression.

2. Système de surveillance (1) selon la revendication précédente, le dispositif de surveillance (3) étant adapté et conçu pour détecter une présence d'une baisse de fuite abrupte à partir du niveau pour le taux de modification, et pour supposer une fuite buccale au moins en fonction d'une présence d'une baisse de fuite abrupte.

3. Système de surveillance (1) selon la revendication précédente, une baisse de fuite abrupte étant présente lorsque le taux de fuite baisse d'au moins 10 % et en particulier d'au moins 20 % dans une période de temps définie.

4. Système de surveillance (1) selon l'une des deux revendications précédentes, une baisse de fuite abrupte étant présente lorsque le taux de fuite baisse d'au moins un niveau défini dans l'intervalle de 8 respirations au maximum et de préférence de 6 respirations au maximum et particulièrement de préférence de 4 respirations au maximum.

5. Système de surveillance (1) selon l'une trois des revendications précédentes, le dispositif de surveillance (3) étant adapté et conçu pour déterminer et sortir un indicateur pour la fréquence de baisses de fuite abruptes par unité de temps et en particulier par heure.

6. Système de surveillance (1) selon l'une des revendications précédentes, le dispositif de surveillance (3) étant adapté et conçu pour détecter une fuite buccale au moins en fonction de savoir si l'au moins un paramètre de fuite varie, et en particulier diminue, d'au moins un niveau défini, dans au moins une période de temps définie, de telle sorte qu'une baisse de fuite abrupte peut être supposée.

7. Système de surveillance (1) selon la revendication précédente, la période de temps définie s'élevant à 30 secondes au maximum et de préférence à 15 secondes au maximum et particulièrement de préférence à 10 secondes au maximum.

8. Système de surveillance (1) selon l'une des deux revendications précédentes, le paramètre de fuite concernant un écoulement d'un flux de gaz concernant la respiration et un niveau défini pour la variation de l'écoulement s'élevant à au moins 10 l/min et de préférence à au moins 15 l/min et particulièrement de préférence à au moins 25 l/min.

9. Système de surveillance (1) selon l'une des revendications précédentes, le dispositif de surveillance (3) étant adapté et conçu pour ne pas prendre en compte une période dans le déroulement temporel du paramètre de fuite pour la détection du type de fuite et/ou ne prendre en compte ladite période que pondérée, lorsque le taux de fuite est encore supérieur à une valeur seuil et en particulier supérieur à 25 l/min, après la baisse de fuite abrupte.

10. Système de surveillance (1) selon l'une des revendications précédentes, le dispositif de surveillance (3) étant adapté et conçu pour ne pas sortir la détection du type de fuite et/ou sortir ladite détection avec au moins un avertissement lorsque le taux de fuite est supérieur ou égal à 50 l/min, en moyenne et/ou sur une période de temps définie.

11. Système de surveillance (1) selon l'une des revendications précédentes, le dispositif de surveillance (3) étant adapté et conçu pour au moins prendre aussi en compte, pour la détection de la fuite buccale, la fréquence avec laquelle des baisses de fuite abruptes se produisent et/ou la régularité avec laquelle des baisses de fuite abruptes se produisent.

12. Système de surveillance (1) selon l'une des revendications précédentes, le dispositif de surveillance (3) étant adapté et conçu pour déterminer l'intervalle de temps entre une baisse de fuite abrupte et une baisse de fuite abrupte précédente et pour au moins aussi prendre en compte la durée de l'intervalle entre deux baisses de fuite abruptes successives, pour la détection de la fuite buccale.

13. Système de surveillance (1) selon l'une des revendications précédentes, le dispositif de surveillance (3) étant adapté et conçu pour prendre en compte au moins aussi des événements de réveil du patient pour la détection de la fuite buccale, et pour prendre en compte un chevauchement temporel entre un événement de réveil et le début d'une baisse de fuite abrupte comme au moins une condition nécessaire pour la présence d'une fuite buccale.

14. Système de surveillance (1) selon l'une des revendications précédentes, le dispositif de surveillance (3) étant adapté et conçu pour utiliser le taux de fuite encore restant après la fermeture d'une fuite buccale détectée pour la détermination d'un taux de fuite souhaité.

15. Système de surveillance (1) selon l'une des revendications précédentes, le dispositif de surveillance (3) étant adapté et conçu pour utiliser une fuite buccale détectée comme au moins une condition nécessaire et/ou suffisante pour la détection d'un événement de réveil du patient.

16. Système de surveillance (1) selon l'une des revendications précédentes, le dispositif de surveillance (3) étant adapté et conçu pour distinguer la fuite de masque au moins par un nombre de baisses de fuite abruptes.

17. Système de surveillance (1) selon l'une des revendications précédentes, le dispositif de surveillance (3) étant adapté et conçu pour effectuer une détection du type de fuite pendant la respiration, à intervalles de temps définis et/ou en continu, et pour consigner et sortir, de préférence par au moins un dispositif d'affichage (5), des types de fuite détectés.

18. Système de surveillance (1) selon l'une des revendications précédentes, le dispositif de surveillance (3) étant adapté et conçu pour effectuer au moins une action en fonction d'un type de fuite détecté, et l'action comprenant une sortie d'un avertissement et/ou une alarme et/ou une description d'action pour éviter les fuites, et le dispositif de surveillance (3) étant adapté et conçu pour commander l'appareil respiratoire (2) en fonction d'un type de fuite détecté et, ce faisant, pour ajuster au moins une grandeur de commande de l'appareil respiratoire (2) afin d'obtenir une diminution du taux de fuite et/ou de la fréquence de fuite.

19. Système de surveillance (1) selon l'une des revendications précédentes, comprenant au moins un appareil respiratoire (2) .
